Europäisches Patentamt

European Patent Office

Office européen des brevets

(19)

(11) **EP 1 216 091 B1**

(12) **FASCICULE DE BREVET EUROPEEN**

(45) Date de publication et mention
de la délivrance du brevet:
**10.03.2004   Bulletin 2004/11**

(51) Int Cl.⁷: **B01J 13/08**, A61K 9/50

(21) Numéro de dépôt: **00958741.1**

(22) Date de dépôt: **25.08.2000**

(86) Numéro de dépôt international:
**PCT/FR2000/002376**

(87) Numéro de publication internationale:
**WO 2001/015799 (08.03.2001 Gazette 2001/10)**

(54) **PROCEDE D'ENCAPSULATION DE MATIERES ACTIVES PAR COACERVATION DE POLYMERES EN SOLVANT ORGANIQUE NON-CHLORE**

VERFAHREN ZUM EINKAPSELN VON AKTIVEN MATERIALIEN DURCH KOAZERVIERUNG VON POLYMERN IN NICHTCHLORIERTEN ORGANISCHEM LÖSUNGSMITTEL

METHOD FOR ENCAPSULATING ACTIVE SUBSTANCES BY COACERVATION OF POLYMERS IN NON-CHLORINATED ORGANIC SOLVENT

(84) Etats contractants désignés:
**AT BE CH CY DE DK ES FI FR GB GR IE IT LI LU
MC NL PT SE**
Etats d'extension désignés:
**AL LT LV MK RO SI**

(30) Priorité: **27.08.1999  FR 9910854**

(43) Date de publication de la demande:
**26.06.2002   Bulletin 2002/26**

(73) Titulaire: **MAINELAB
49100 Angers (FR)**

(72) Inventeurs:
• **BENOIT, Jean-Pierre
F-49240 Avrille (FR)**
• **RICHARD, Joel
F-49160 Longue (FR)**
• **FOURNIER, Elvire
F-49100 Angers (FR)**
• **LIU, Sonia
28170 Maillebois (FR)**

(74) Mandataire: **Ahner, Francis et al
Cabinet Régimbeau
20, rue de Chazelles
75847 Paris cedex 17 (FR)**

(56) Documents cités:
**EP-A- 0 330 180         WO-A-95/13799
WO-A-97/41837         US-A- 5 324 520**

• **THOMASIN C ET AL: "DRUG
MICROENCAPSULATION BY PLA/PLGA
COACERVATION IN THE LIGHT OF
THERMODYNAMICS. 1. OVERVIEW AND
THEORETICAL CONSIDERATIONS" JOURNAL
OF PHARMACEUTICAL
SCIENCES,US,AMERICAN PHARMACEUTICAL
ASSOCIATION. WASHINGTON, vol. 87, no. 3, 1
mars 1998 (1998-03-01), pages 259-268,
XP000736341 ISSN: 0022-3549**

Remarques:
Le dossier contient des informations techniques présentées postérieurement au dépôt de la demande et ne figurant pas dans le présent fascicule.

**Description**

La présente invention concerne un procédé de microencapsulation d'un principe actif par coacervation, utilisé notamment pour préparer des formes galéniques à libération prolongée.

Les techniques de microencapsulation sont classiquement utilisées pour séparer des substances chimiques incompatibles, convertir des liquides en poudres, améliorer la biodisponibilité de principes actifs, masquer le goût ou l'odeur désagréable de certains composés et préparer des formes galéniques à libération prolongée.

Les formes galéniques à libération prolongée sont susceptibles d'être administrées par voie sous-cutanée ou intramusculaire, et de se retrouver directement dans le flux sanguin ou à proximité de l'organe à traiter si bien que les polymères biodégradables sont souvent choisis pour entrer dans leur composition.

Les systèmes à libération prolongée à base de polymères biodégradables peuvent être administrés par la voie parentérale, sans retrait par intervention chirurgicale, puisque les polymères biodégradables se transforment dans l'organisme en métabolites éliminés par les voies naturelles. Le principe actif est libéré, suivant une cinétique modulée par la diffusion du principe actif et le processus de dégradation du polymère. De plus, l'observance du patient est améliorée du fait d'une administration moins fréquente.

Parmi les polymères biodégradables d'emploi fréquent dans l'encapsulation de principes actifs figurent les poly($\alpha$-hydroxy-acides), notamment les polymères d'acide lactique (PLA) et les polymères d'acides lactique et glycolique (PLAGA), la poly-$\varepsilon$-caprolactone, les polyorthoesters comme Chronomer® et Alzamer®, les polyanhydrides, notamment le copolymère d'acide sébacique et de (carboxyphénoxy)propane, les polypeptides et les polymères naturels biodégradables, comme l'albumine, la sérum albumine bovine, le collagène et le chitosane.

Il existe deux grands types de techniques de microencapsuiation :

- les techniques sans solvant, comme le spray-congealing, l'extrusion (coextrusion/sphéronisation), la gélification, le prilling et la précipitation de solutions supercritiques (RESS), et
- les techniques avec solvants, comme la nébulisation, la coacervation, l'émulsion-évaporation, l'émulsion-extraction, avec leurs variantes à partir d'émulsions doubles eau/huile/eau.

Le contact prolongé des formes galéniques à libération prolongée avec un milieu aqueux justifie l'emploi de polymères à caractère plus ou moins hydrophobe, donc principalement solubles dans un milieu organique. Les polymères biodégradables à la base de systèmes à libération contrôlée sont cependant faiblement solubles dans les solvants de faible toxicité potentielle (solvants de classe 3 selon la norme ICH).

C'est pourquoi, les techniques classiques de microencapsultation (coacervation, émulsion-évaporation) emploient essentiellement des solvants chlorés comme le dichlorométhane (solvant de classe 2 selon la norme ICH, i.e. solvant à limiter) comme solvant du polymère. Or c'est un solvant chloré connu pour sa neurotoxicité. Le taux résiduel de dichlorométhane autorisé dans le produit fini est de 600 ppm selon la norme ICH4.

Quelle que soit la technique de microencapsulation employée, les microparticules obtenues renferment des quantités de solvants résiduelles. Il apparaît donc nécessaire de développer de nouvelles méthodes de microencapsulation ne faisant pas appel aux solvants chlorés. Deux solutions majeures se présentent pour résoudre ce problème.

Une solution permettant de réaliser l'encapsulation sans solvants chlorés repose sur des méthodes ne mettant en oeuvre aucun solvant, mais certains polymères ne peuvent pas être mis en oeuvre selon ces méthodes. En outre, les propriétés des particules obtenues par ces méthodes ne correspondent pas forcément aux exigences d'un traitement à long terme.

Une autre solution consiste à substituer les solvants chlorés par des solvants non toxiques. Les méthodes de microencapsulation qui utilisent des solvants chlorés ont été largement étudiées et les facteurs de variation sont connus. Cependant, la substitution des solvants chlorés par des solvants non chlorés modifient les interactions physicochimiques entre les différents composants de la formulation. Le comportement des polymères biodégradables dans les solvants de substitution est très différent de celui dans les solvants chlorés. Ainsi, le poly(L-lactide) et le poly(D,L-lactide) sont insolubles dans l'acétate d'éthyle ou l'acétone et aucun polymère n'est soluble dans l'alcool éthylique, qui est pourtant un solvant de prédilection puisqu'il est faiblement toxique.

La présente invention propose un procédé de microencapsulation par coacervation qui n'utilise aucun solvant chloré. Plus précisément l'invention concerne un procédé de coacervation par ajout de non-solvant. La coacervation par ajout de non-solvant requiert l'usage de trois solvants miscibles ; un de ces trois solvants est solvant du polymère et les deux autres sont non-solvants du polymère.

Le principe de la coacervation repose sur la désolvatation ménagée d'un polymère dissous dans un solvant organique contenant un principe actif généralement sous forme particulaire, induit par addition d'un non-solvant ou agent de coacervation du polymère. La solubilité du polymère dans le solvant organique est abaissée et il se forme deux phases non miscibles : le coacervat se dépose progressivement à la surface du principe actif. L'ajout d'un durcisseur permet la formation d'un film continu de polymère autour du principe actif.

Les particules de principe actif peuvent être liquides ou solides. Le principe actif peut également être initialement dissous dans le solvant du polymère. Il reprécipite alors sous forme particulaire lorsque l'on ajoute l'agent de coacervation, ou peut former une solution solide homogène dans les particules de polymère issues de la coacervation.

L'étude des interactions polymère/solvant/agent de coacervation, pour chaque association, permet de réaliser un diagramme de phases de façon à définir le rapport idéal polymère/solvant/agent de coacervation nécessaire à une encapsulation efficace. Cependant, il est difficile de prédire l'encapsulation d'un principe actif, car les propriétés interfaciales, en rapport avec les interactions moléculaires entre le polymère, le solvant et l'agent de coacervation, changent constamment avec la composition du coacervat (Thomassin C., Merlcle H.P., Gander B.A., Physico-chemical parameters goveming protein microencapsulation into biodegradable polyester by coacervation, Int. J. Pharm, 1997, 147, 173-186).

Le problème majeur de la technique de coacervation est l'agglomération possible de particules. Pour tenter de le résoudre, des auteurs ont proposé de réduire la température du système, essentiellement à l'étape de durcissement. Les parois sont alors suffisamment solides pour éviter l'adhésion. Des solutions telles que l'emploi de chlorofluoro-carbones (CFC) ou l'abaissement de la température ne sont pas transposables à l'échelle industrielle. En revanche, l'emploi d'huile de silicone est capable de stabiliser le système grâce, à sa viscosité (Ruiz J.M., Tissier B., Benoit J.P., Microencapsulation of peptide : a study of the phase séparation of poly(D,L-lactic acid-co-glycolic acid) copolymers 50/50 by silicone oil, Int. J. Pharm., 1989, 49, 69-77).

La technique de coacervation, malgré des taux élevés de solvants résiduels reste une technique de choix pour l'encapsulation de principes actifs fragiles, et surtout de principes actifs hydrosolubles, en milieu non aqueux.

Le choix des associations solvant/agent de coacervation/durcisseur est guidé par divers critères :

- le solvant doit solubiliser le polymère ; on préfère qu'il ne solubilise pas le principe actif, bien que le procédé reste toujours utilisable avec un principe actif soluble dans le solvant du polymère.
- l'agent de coacervation doit être miscible au solvant du polymère. il ne doit pas être solvant du polymère, car sinon cela reviendrait à un simple transfert du polymère du solvant vers l'agent de coacervation. De plus, il ne doit pas solubiliser le principe actif pour limiter les pertes d'encapsulation.
- le durcisseur doit être partiellement miscible au solvant du polymère pour en faciliter l'extraction. Il ne doit solubiliser, ni le polymère, ni le principe actif, car sinon le rendement d'encapsulation serait fortement diminué.

Dans l'art antérieur, la technique de coacervation fait appel au dichlorométhane ou au chloroforme comme solvant du polymère, à une huile silicone comme agent de coacervation, et à l'heptane comme durcisseur.

La présente invention concerne un procédé de microencapsulation d'un principe actif par coacervation qui consiste en

- la désolvatation ménagée ou coacervation d'un polymère dissous dans un solvant organique contenant ledit principe actif, ladite coacervation étant induite par addition d'un non-solvant et se traduisant par le dépôt du polymère à la surface dudit principe actif, puis
- le durcissement du dépôt de polymère par ajout d'un agent durcisseur, ledit durcissement se traduisant par la formation d'un film continu enrobant ledit principe actif,

caractérisé en ce que

- le solvant du polymère est un solvant organique non chloré de point d'ébullition compris entre 30°C et 240 °C et de permittivité diélectrique relative et comprise entre 4 et 60, avantageusement choisi parmi l'acétate d'éthyle, la N-méthylpyrrolidone, la méthyléthylcétone, l'acide acétique, le propylène carbonate et leurs mélanges,
- le non-solvant est un alcool ou une cétone comprenant 2 à 5 atomes, et préférentiellement 2 ou 3 atomes de carbone, en particulier l'éthanol ($\varepsilon$=24), le propan-2-ol ($\varepsilon$=18), le propane-1,2-diol ($\varepsilon$ entre 18 et 24) et le glycérol ($\varepsilon$=40), ou la méthyléthylcétone ($\varepsilon$= 18),
- l'agent durcisseur est choisi parmi l'eau, les alcools comprenant 1 à 4 atomes de carbone à condition que l'agent durcisseur soit un alcool différent du non-solvant, et leurs mélanges.

Bien que la N-méthylpyrrolidone soit de classe 2 comme le dichlorométhane, sa concentration limite est nettement plus élevée (4840 ppm contre 600 ppm pour le dichlorométhane).

De façon avantageuse, le non-solvant et le durcisseur sont respectivement choisis parmi les couples suivants : propane-1,2-diol et propan-2-ol, glycérol et propane-1,2-diol, glycérol et propan-2-ol, propan-2-ol et propane-1,2-diol.

Selon un mode de réalisation préféré, le polymère est un polymère biodégradable dont la masse moléculaire en poids (Mw) est comprise entre 10 000 et 90 000 g/mole, de préférence entre 15 000 et 50 000 g/mole, dont l'indice de polydispersité (Ip) est comprise entre 1 et 3,5, de préférence entre 1,5 et 2,5.

On indiquera ci-après un certain nombre d'autres caractéristiques additionnelles pour illustrer divers modes de mises en oeuvre préférentielles de l'invention.

Selon ces caractéristiques additionnelles, le polymère est un polymère d'acide lactique (PLA) ou un copolymère d'acides lactique et glycolique (PLAGA).

Le polymère est un PLAGA tel que Mw est comprise entre 15 000 et 20 000 g/mole, de préférence égal à 17 500, Ip est compris entre 1 et 2, de préférence égale à 1,6, et le pourcentage d'acide glycolique est inférieur à 30%, de préférence égal à 25 %.

La concentration en polymère dans le solvant est comprise entre 1 et 10% (p/v), de préférence de l'ordre de 4% (p/v).

Le rapport volumique non-solvant/solvant est compris entre 1/2 et 1/1.

La température de coacervation est inférieure à la température de transition vitreuse du polymère, de préférence inférieure ou égale à 25°C, de préférence inférieure à 4°C, de préférence encore inférieure ou égale à -4°C.

Le non-solvant est ajouté par doses successives de 200μl à 1ml.

La coacervation est réalisée sous agitation, par exemple sous agitation magnétique, à une vitesse comprise entre 200 et 1000t/min.

L'agent durcisseur contient en outre un agent tensioactif, la concentration dudit agent tensioactif dans l'agent durcisseur étant comprise entre 0,1 et 10% (v/v) en particulier entre 0,5 et 10% (v/v).

L'agent tensioactif est un ester de sorbitan, par exempte le Tween® 80, ou l'alcool polyvinylique.

Le rapport volumique agent durcisseur/solvant est compris entre 5/1 et 180/1, et de préférence entre 15/1 et 120/1.

Le durcissement des microsphères est réalisé sous agitation, par exemple magnétique à une vitesse comprise entre 500 et 1500 t/min.

La température de durcissement est inférieure ou égaie à 25°C, de préférence inférieure à 4°C, de préférence encore inférieure ou égale à 0,5°C.

Le durcisseur est ajouté en plusieurs fois, de préférence en quatre fois au minimum.

Le durcissement dure entre 2 et 4 heures.

Les microparticules obtenues à la suite du durcissement sont filtrées sur système Millipore®, par centrifugation ou sur papier plissé.

Lorsque le principe actif forme une dispersion dans la solution de polymère, le solvant et le non-solvant ont une viscosité suffisamment élevée pour stabiliser le principe actif.

La granulométrie du principe actif est comprise entre 1 et 50 microns, et préférentiellement entre 5 μm et 30 μm.

Selon un mode de réalisation préféré, le solvant est la N-méthylpyrrolidone, le non-solvant est l'éthanol, et le durcisseur est l'eau.

Selon un autre mode de réalisation préféré, le solvant est l'acétate d'éthyle, le non-solvant est le propan-2-ol, et le durcisseur est l'eau. Le polymère est un PLAGA 75 :25 tel que Mw est comprise entre 15 000 et 20 000, de préférence égal à 17 500, Ip est compris entre 1 et 2, de préférence égal à 1,6.

Selon un troisième mode préféré de réalisation, le solvant est l'acide acétique, le durcisseur est l'eau, et le polymère est un PLAGA 50 :50.

Dans le cadre de la présente invention, lorsque le principe actif est insoluble dans le solvant du polymère, on réalise soit une suspension, soit une émulsion.

Pour préparer la suspension, le principe actif est broyé au mortier puis mis en suspension dans le solvant. La suspension peut être homogénéisée sous agitation magnétique : la coacervation est alors aussi réalisée sous agitation magnétique. La suspension peut également être homogénéisée sous agitation mécanique, à vitesse variable (agitateur à hélice, Heidolph RGL500, Prolabo, Paris, France) ou à l'aide d'un Ultra-Turrax® T25 (Prolabo, Paris, France). Dans ces deux derniers cas, la coacervation est alors réalisée sous agitation mécanique.

La dispersion du principe actif dans la solution de polymère peut également être classiquement réalisée sous agitation par ultrasons.

Ainsi, le principe actif peut être dispersé sous ultrasons pour former une dispersion dans la solution de polymère, et la coacervation peut être réalisée sous agitation douce, de préférence de type magnétique ou mécanique.

Lorsque le principe actif est hydrosoluble, pour préparer l'émulsion, le principe actif est dissous dans l'eau, puis une émulsion eau/solvant du polymère est réalisée sous agitation mécanique. La coacervation se fait ensuite sous agitation mécanique.

Lorsque le principe actif est soluble dans le solvant du polymère, la coacenration est réalisée sous agitation mécanique.

Dans le cadre de la présente invention, le polymère est un polymère biodégradable d'emploi fréquent dans l'encapsulation de principes actifs, de préférence un PLA ou un PLAGA, de préférence encore un PLAGA de masse molaire en poids (Mw) comprise entre 10 000 et 90 000, de masse molaire en nombre (Mn) comprise entre 4 0000 et 40 000, d'indice de polydispersité (Ip) compris entre 1 et 3,5 et dont la proportion de glycolide est comprise entre 10 et 60%. On préférera les polymères de masse molaire en poids élevée supérieure ou égale à 15 000 g/mole car ils permettent d'augmenter le rendement de fabrication en augmentant le volume de la phase de coacervat. On préférera également les polymères dont l'indice de polydispersité est faible (Ip ≤ 2,6), car les fractions de bas poids moléculaire restent en solution et entraînent une baisse de rendement, ou provoquent l'agglomération des microparticules en se collant à leur surface.

Le PLAGA est par exemple le Resomer® RG 502 (Boehringer Ingelheim, Mw=14 300 g/mole), Mn 6 900 g/mole, Ip 2,5, 50 % de glycolide), le Resome® RG 756 (Mw 89 800 g/mole, Mn=35 200 g/mole, Ip 2,6, 25 % de glycolide), le Resomer® RG 858 (Mw=87 000 g/mole, Mn=22 000 g/mole, Ip 3,9, 15 % de glycolide), le Phusiline foumi par Phusis (Mw=17 500 g/mole, Mn =10 940 g/mole, Ip=1,6, 25 % de glycolide).

La concentration en polymère dans le solvant doit être suffisante pour augmenter la viscosité du milieu, ce qui permet de stabiliser les gouttelettes de coacervat dispersées et de limiter leur agrégation, d'une part, et de diminuer la formation de microparticuies de petite taille, d'autre part.

La concentration en polymère est de préférence comprise entre 1 et 10 % (p/v), de préférence encore égale à environ 4 % (p/v).

Les viscosités du solvant et du non-solvant doivent être suffisantes pour stabiliser les gouttelettes de coacervat.

Le volume de non-solvant à ajouter est défini de manière à porter le système dans la fenêtre de stabilité et obtenir un coacervat stable. Cependant, le volume de non-solvant dépend également de la concentration des cristaux de principe actif en suspension dans la solution organique de polymère.

L'addition d'un excès de non-solvant permet d'accélérer le durcissement de la paroi des microparticules, de prévenir leur agglomération et d'améliorer l'extraction du solvant.

La vitesse d'addition du non-solvant est suffisamment basse pour éviter la formation d'un grand nombre de microparticules trop petites, i.e. de taille comprise entre 1 et 2 μm. En outre, plus la séparation de phase est lente, plus la distribution granulométrique des microparticules est uniforme et plus la surface des microparticules est lisse. L'ajout de non-solvant se fait de préférence graduellement par doses de 200 μl à 1 ml, en observant au moins une minute entre chaque dose.

Une diminution de la vitesse d'agitation pendant l'étape de coacervation augmente la taille des gouttelettes de coacervat puis des microparticutes finales. Mais en dessous d'une vitesse limite, variable en fonction des systèmes, la cinétique de dépôt du coacervat est trop lente et/ou les gouttelettes de coacervat sont trop grosses et ne sont pas assez stables. Une agitation mécanique ou magnétique comprise entre 200 t/min et 1000 t/min donne souvent de bons résultats.

La température est le paramètre essentiel de la coacervation ; elle doit être inférieure à la température de transition vitreuse du polymère. Plus elle est basse, plus le milieu est visqueux et moins les microparticules ont tendance à s'agréger.

Le durcisseur idéal ne doit solubiliser ni le principe actif, ni le polymère. Il doit extraire facilement le solvant du polymère. Le durcisseur employé est de l'eau éventuellement additionnée de tensioactif ou d'un alcool. L'eau permet avantageusement d'extraire facilement le solvant du polymère. Elle présente, en outre, l'avantage d'être de faible coût, de ne pas nécessiter de retraitement des effluents. Cependant, l'eau n'est pas le durcisseur idéal, dans le cas de principes actifs hydrosolubles car tout contact prolongé est responsable de la diffusion du principe actif qui se traduit par un faible taux d'encapsulation.

Lorsque le principe actif encapsulé est hydrophile pendant le durcissement, il est rapidement dissous par l'eau qui pénètre dans les microparticules, et peut diffuser en retour hors des particules. En travaillant à basse température, on diminue les phénomènes de diffusion, donc les fuites de principe actif vers la phase aqueuse, et on améliore le rendement d'encapsulation.

D'autres possibilités peuvent être envisagées pour réduire la diffusion du principe actif ; comme la saturation de la phase externe par un électrolyte ou le principe actif lui-même, s'il est de faible coût, et l'association de l'eau à un autre solvant, qui présente une forte affinité pour le solvant du polymère afin de l'extraire des microsphères. Ainsi, on réduit le volume d'eau employé et le contact avec l'eau est atténué.

Le tensioactif ou l'alcool permettent de limiter l'agrégation des microparticules entre elles afin de former une dispersion homogène. Ils ont été sélectionnés en fonction de leur innocuité. Les tensioactifs sont choisis parmi ceux fréquemment utilisés dans les formulations destinées à la voie injectable tels que les esters de sorbitan polyoxyéthylénés, comme le Tween® 80 (Polysorbate 80) et le Tween® 20 (Polysorbate 20) (tensioactifs hydrophiles).

Le Montanox® 80 (monooléate de sorbitan polyoxyéthyléné) est un émulsifiant hydrophile, qui peut entrer dans la composition d'une émulsion de type Huile/Eau.

Le Montane® 80 (oléate de sorbitane) est son équivalent dans la gamme des tensioactifs lipophiles. Le Solutol® HS 15 (hydroxystéarate de polyéthylène glycol 660) est un tensioactif non ionique, à caractère hydrophile utilisé dans les solutions injectables.

Le Synperonic® PE/F 68 (Poloxamer 188) est un copolymère-bloc de polyoxyéthylène et de polyoxypropylène.

Enfin, l'alcool polyvinylique, a été employé sous deux grades différents : le Mowiol® 4/88 et le Rhodoviol®4/125.

Le volume de durcisseur relève d'un compromis. Il doit être suffisant pour éliminer rapidement le solvant des microsphères, mais il devra limiter la diffusion du principe actif hors des microsphères. Le volume est défini d'après les critères de solubilité du solvant dans la phase externe, de manière à ce que la concentration finale du solvant dans le durcisseur soit inférieure à la concentration à saturation de la phase externe en solvant. En éliminant rapidement le solvant, la diffusion du principe actif est empêchée par la formation d'une barrière de polymère. De plus, les microsphères seront durcies ce qui évite l'agrégation.

L'augmentation du volume du durcisseur, faite de façon progressive par ajouts à intervalles réguliers, permet de mieux extraire le solvant des microparticules.

Le rapport du volume du durcisseur au volume du solvant est compris entre 5/1 et 180/1, et de préférence entre 15/1 et 120/1.

Les associations utilisant l'acide acétique ou la N-méthylpyrrolidone comme solvant du polymère nécessitent peu d'agent durcisseur pour donner des microsphères solides, si bien que le rapport du volume du durcisseur au volume du solvant est avantageusement de l'ordre de 5/1 dans ce cas.

La méthode de séchage des microparticules est fonction de la rigidité des microsphères, de la taille et des volumes à traiter.

La tendance des microparticules à s'agréger après séchage dépend de leur taux d'hydratation et des quantités de solvant résiduelles.

Si un séchage à l'air libre est insuffisant, la mise sous vide et/ou l'augmentation de la température permettent de la compléter, à condition que les microparticules soient résistantes au vide. Il faut cependant s'assurer que la vitesse et la température de séchage ne soient pas trop élevées pour éviter l'agglomération des microparticules.

Dans le cas des associations utilisant l'acétate d'éthyle comme solvant du polynère, se pose le problème du choix de la méthode de séparation des microsphères. En effet, les microsphères sont encore gorgées de solvant après une agitation d'une heure dans le durcisseur. Une filtration sur un système Millipore®, à travers un filtre de porosité 0,5 μm donne lieu à un gâteau solide difficilement redispersable. De plus, le filtre est très vite colmaté par les microsphères encore déformables. La séparation par centrifugation sur un aliquot de la solution n'a pas donné le résultat espéré. Là encore, les microsphères s'agrègent et forment un culot, non redispersable, même à une vitesse de centrifugation peu élevée. Une autre technique de filtration utilisant un filtre en papier plissé, s'avère être une solution. Ce procédé présente l'avantage d'une surface de filtration importante et se réalise à pression atmosphérique. Cependant, les microsphères les plus petites ont tendance également à s'adsorber dans les pores et colmatent le filtre au fur et à mesure. Il est également difficile de récupérer la totalité des microsphères. Quant au séchage, il est assuré par un courant d'air comprimé, puis les microsphères sont laissées à l'air ambiant.

Selon un mode de réalisation de l'invention, le solvant du polymère est l'acétate d'éthyle, le non-solvant est le propan-2-ol, et le durcisseur est un mélange eau/tensioactif, éventuellement un mélange eau/tensioactif/alcool.

Dans ce mode de réalisation, le polymère est de préférence un PLAGA 75 : 25. La concentration en polymère est comprise entre 1 et 5 % (p/v), de préférence égaie à environ 4 % (p/v). La coacervation est menée à température ambiante, de préférence à une température inférieure à 4°C, de préférence encore inférieure à égale à - 4°C, sous agitation mécanique, de préférence à 300 t/min.

La concentration en tensioactif est comprise entre 1 et 10 % (v/v). Le tensioactif est le Tween® 80. Lorsqu'un alcool est associé à de l'eau, on fixe la concentration en tensioactif entre 1 et 10 % environ et la concentration en alcool entre 2,5 et 5 % environ. L'alcool est avantageusement le propan-2-ol ou le propane-1,2-diol. La solution aqueuse de durcisseur est ajoutée en au moins quatre fois. Le durcissement dure au moins 2h30 et au plus 4 heures, il est effectué à température ambiante, de préférence à une température inférieure à 4°C, de préférence encore à 0,5°C, sous agitation mécanique (500 t/min).

Le rapport volumique non-solvant /solvant est égal à 1/2.

Le rapport volumique durcisseur/solvant est égal à 120/1.

Plus la température de durcissement est basse, plus la durée de durcissement sera diminuée. Ainsi, lorsque la température est inférieure à 4°C, une durée de 4 heures suffit. La durée est abaissée à 2h30 quand la température est de 0,5°C.

Selon un autre mode de réalisation, le solvant est la N-méthylpyrrolidone, le non-solvant est l'éthanol et le durcisseur est un mélange eau/tensioactif. La concentration en polymère est comprise entre 4 et 10 % (p/v). La coacervation et le durcissement sont conduits à température ambiante, sous agitation magnétique. La concentration en tensioactif est comprise entre 0,5 et 10 % (v/v). La durée de durcissement est comprise entre 2 et 4 heures. Le rapport volumique durcisseur/solvant est égal à 40/1. Le polymère est de préférence le Résomer RG® 502 ou le résomer RG® 756.

La présente invention est illustrée par les exemples suivants sans en limiter la portée.

**<u>Exemple 1</u> : Evaluation des associations polymère/solvant/agent de coacervation/durcisseur.**

Dans cette étude, les poly($\alpha$-hydroxy-acides) ont été évalués. Trois copolymères d'acide lactique et glycolique, dont les proportions de L-, D-lactides et glycolides sont variables, ont été employés. Il s'agit des polymères suivants, fournis par Boehringer Ingelheim :

- le Resomer® RG 502 (Mw=14300g/mole, Mn=6900g/mole), qui comporte 25 % de L-lactide, 25 % de D-lactide et 50 % de glycolide,
- le Resomer® RG 756 (Mw=89800g/mole, Mn=35200g/mole), qui renferme 37,5 % de L-lactide, 37,5 % de D-lactide et 25 % de glycolide,
- le Resomer® RG 858 (Mw=87000 g/mole, Mn=22000 g/mole), qui possède 42,5 % de L-lactide, 42,5 % de D-lactide et 15 % de glycolide.

Au vu des solvants respectifs des polymères, les solvants les moins toxiques ont été sélectionnés. Ils appartiennent aux solvants de classe 2 ou 3, définis par la classification des lignes directrices de l'ICH.

La miscibilité des couples solvant/non-solvant des trois polymères étudiés a été ensuite déterminée. De la même manière, les non-solvants des polymères ont été choisis pour leur faible toxicité.

Le criblage des associations solvant/non-solvant/durcisseur est réalisé dans des flacons à scintillation, sur de faibles volumes de solutions organiques de polymères à 1 ou 4 (pour la N-méthylpyrrolidone et le PLAGA 50 : 50 exclusivement) % (m/v), 5 ml de solution organique de polymère sont placés dans un flacon à scintillation. On ajoute ensuite l'agent de coacervation jusqu'à obtenir un trouble, persistant à l'agitation, qui est caractéristique de la formation de coacervat. Le coacervat est observé à cette étape en microscopie optique. Puis 1 ml de ce mélange est versé dans 10 ml d'une solution aqueuse de tensioactif. On observe par microscopie optique la présence ou non de microsphères.

**a) Essais de coacervation avec le PLAGA 50 : 50 (Resomer® RG 502)**

Les solvants dans lesquels le PLAGA 50 : 50 est soluble sont l'acétate d'éthyle, l'acétone, l'acétonitrile, l'acide acétique, le diméthylacétamide, le diméthylformamide, le lactate d'éthyle, la N-méthylpyrrolidone et le propylène carbonate. Le PLAGA 50 : 50 est insoluble dans le toluène, le propan-2-ol, le glycérol, le dioctyladipate, le propane-1,2-diol, le xylène, le diéthylcarbonate et la méthyléthyicétone.

Les associations pour lesquelles on observe la formation d'un coacervat et de microsphères sont les suivants :

- acétate d'éthyle/propan-2-ol/eau + Tween® 80,
- acide acétique/propane-1,2-diol ou propan-2-ol /eau + Tween® 80,
- N-méthylpyn-olidone/éthanol/eau + Tween® 80 ou propan-2-ol,
- N-méthylpyrrolidone/méthyléthylcétone ou propan-2-ol/eau + Tween®80.
- Propylène carbonate/propan-2-ol/ eau éventuellement avec Tween® 80, éthanol ou NaCl.

L'acide acétique peut être employé comme solvant du polymère et donner lieu à la formation de coacervat et de microsphères bien individualisées, à condition d'utiliser l'eau comme durcisseur. Effectivement, il se forme des gouttelettes de coacervat dans l'association, acide acétiquelpropan-2-ol mais en présence de méthyléthytcétone ou propane-1,2-diol comme durcisseurs, les gouttelettes de coacervat ne conservent pas leur forme sphérique et il se forme des amas de polymères.

L'association acétate d'éthyle/propan-2-olleau + Tween® 80 donne de bons résultats ainsi que l'association N-méthylpyrrotidoneléthanol/eau + Tween® 80. Cette étude a été complétée par la recherche de durcisseurs. Le propane-1,2-diol et le propan-2-ol s'avèrent être de bons candidats. Cependant, on peut noter une capacité d'extraction du solvant moindre avec le propane-1,2-diol qu'en présence de propan-2-ol.

Le propylène carbonate est un solvant intéressant par sa faible toxicité mais aussi par sa solubilité partielle dans l'eau. L'association propylène carbonatelpropan-2-ol permet de s'affranchir de l'eau grâce à la possibilité de remplacer la phase externe par un solvant ou du moins, limiter la diffusion du principe actif, par ajout d'un électrolyte dans la phase externe ou par mélange d'eau et d'un solvant.

**b) Essais de coacervation avec le PLAGA 75 : 25 (Resomer® RG 756)**

Les solvants dans lesquels le PLAGA 75 : 25 est soluble sont l'acétate d'éthyle, l'acétone, l'acétonitrile, l'acide acétique, le diméthytacétamide, le diméthylformamide, l'éther diéthylique, la méthyléthylcétone, et la N-méthylpyrrolidone.

Le PLAGA 75: 25 est insoluble dans le toluène, le propan-2-ol, le glycérol, le propane-1,2-diol, le propylène carbonate, le dioctyladipate et le triéthylcitrate.

Les deux solvants donnant les meilleurs résultats sont l'acide acétique et l'acétate d'éthyle. Les études se sont donc focalisées sur l'évaluation des associations acide acétique/glycérol et acide acétique/propane-1,2-diol ainsi que les couples acétate d'éthyle/propan-2-ol et acétate d'éthyle/propane-1,2-diol.

Les associations pour lesquelles on observe la formation de microsphères individualisées sont :

- acétate d'éthyle/propane-1,2-diol/eau + Tween® 80 ou propan-2-ol,
- acide acétique/glycérol ou propane-1,2-diol/eau + Tween® 80.

### c) Essais de coacervation avec le PLAGA 85 : 15 (Resomer® RG 858)

Les solvants dans lesquels le PLAGA 85 : 15 est soluble sont l'acétate d'éthyle, l'acétone, l'acétonitrile, l'acide acétique, la diméthylformamide, l'éthanolamine, l'éthylènediamine, la méthyléthylcétone, la N-méthylpyrrolidone, le toluène et le triéthylcitrate. Le PLAGA 85 : 15 est insoluble dans le propan-2-ol, le glycérol, le propane-1,2-diol, le dioctyladipate et le xylène.

Les associations pour lesquelles on observe la formation de coacervat et de microsphères sont :

- acétate d'éthyle/propan-2-ol ou propane-1,2-diol/eau + Tween® 80,
- acétate d'éthyle/propan-2-ol/propane-1,2-diol,
- acétate d'éthyle/propane-1,2-diol/propan-2-ol,
- acide acétique/glycérol (eau + Tween® 80) ou propane-1,2-diol ou propan-2-ol,
- acide acétique ou méthyléthylcétone/propane-1,2-diol/eau + Tween® 80,
- acide acétique/propan-2-ol/propane-1,2-diol,
- N-méthylpyrrolidone ou méthyléthylcétone/propan-2-ol/eau + Tween® 80,
- méthyléthylcétone/propane-1,2-diol/propan-2-ol.

### Exemple 2 : Réalisation de microsphères non chargées en principe actif en faisant varier les paramètres de la coacervation.

Dans un premier temps les conditions opératoires sont établies pour la préparation de microsphères sans principe actif, afin de réaliser des particules de la taille désirée. L'influence de différents facteurs comme le volume d'agent de coacervation ajouté, le volume de durcisseur, le type et la vitesse d'agitation, et la méthode de collecte des microsphères est étudiée.

Le polymère est dissous dans 50 ml de solvant organique (Bécher n° 1) pour donner une solution à 1 % (p/ v). La concentration en polymère est portée à 4 % (p/v) quand le solvant est la N-méthylpyrrolidone. Sous agitation, on ajoute l'agent de coacervation jusqu'à l'obtention d'un coacervat stable et visible. Puis le mélange est versé dans une solution de durcisseur additionnée d'un tensjoactif (Bécher n° 2), sous agitation. Les microsphères sont ensuite récupérées par filtration. Les associations testées sont celles sélectionnées dans l'exemple 1.

### a) avec le PLAGA 50 : 50 (Resomer® RG 502).

Les différents protocoles correspondant à chacune des associations retenues sont récapitulés dans le tableau 1.

**TABLEAU 1**

| Modes opératoires définis pour les formulations de microsphères non chargées en principe actif à base de PLAGA 50 : 50 | | | | | | | | |
|---|---|---|---|---|---|---|---|---|
| Associations Solvant/non-solvant (avec eau + tensioactif comme durcisseur) | Volume de non-solvant | Agitation Bécher 1 | Vitesse Bécher 1 | Agitation Bécher 2 | Vitesse Bécher 2 | Volume de durcisseur | Méthode de filtration | Taille des micro-sphères |
| Acétate d'éthyle/ propan-2-ol | 9 ml | Magnétique | 500 t/min | magnétique | 700 t/min | 750 ml | Filtre plissé | 40 µm |
| Acide acétique/ Propane-1,2-diol | 16 ml | Ultrasons | | magnétique | 1100 t/min | 250 ml | Filtre plissé | 24 µm |
| Acide acétique/ propan-2-ol | 16 ml | Ultrasons | | magnétique | 1400 t/min | 250 ml | Filtre plissé | 67 µm |
| N-méthylpyrrolidone/ Ethanol | 31 ml | Mécanique | 200 t/min | magnétique | 1000 t/min | 1000 ml | Centrifu-gation | 31 µm |
| N-méthylpyrrolidone/ propan-2-ol | 40 ml | Magnétique | 300 t/min | magnétique | 1000 t/min | 500 ml | Filtre plissé | 70 µm |

**b) Application au PLAGA 75 : 25 (Resomer® RG 756).**

Des microsphères sont obtenues dans les conditions décrites dans le tableau 2 avec l'acétate d'éthyle comme solvant. Apparaissent trois possibilités d'agitation pour l'association acétate d'éthyle/propane-1,2- diol/eau + Montanox® 80 ou Tween® 80 : l'agitation par les ultrasons, l'agitation mécanique à pales et l'agitation magnétique. Il n'y a pas de différences notables dans la morphologie ou la taille des microsphères qui puissent justifier le choix de l'une de ces méthodes.

## TABLEAU 2

| Modes opératoires définis pour les formulations de microsphères non chargées en principe actif à base de PLAGA 75 : 25 | | | | | | | | |
|---|---|---|---|---|---|---|---|---|
| Associations Solvant/non-solvant (avec eau + tensioactif comme durcisseur) | Volume de non-solvant | Agitation Bécher 1 | Vitesse bécher 1 | Agitation Bécher 2 | Vitesse Bécher 2 | Volume de durcisseur | Méthode de filtration | Taille des micro-sphères |
| Acétate d'éthyle/ Propane-1,2-diol | 15 ml | ultrasons | | magnétique | 1100 t/min | 750 ml | Filtre plissé | 20 µm |
| Acétate d'éthyle/ Propane-1,2-diol | 15 ml | mécanique | 400 t/min | magnétique | 1100 t/min | 750 ml | Filtre plissé | 25 µm |
| Acétate d'éthyle/ propan-2-ol | 16 ml | magnétique | 900 t/min | magnétique | 1100 t/min | 800 ml | Filtre plissé | 32 µm |

EP 1 216 091 B1

**Exemple 3 : Réalisation de microsphères chargées en 5-fluorouracile (5-FU) comme principe actif.**

Les essais sont réalisés avec les associations de l'exemple 1 a) et b) qui ont conduit à l'observation d'un coacervat.

**a) Influence du principe actif sur la coacervation.**

Le principe actif à faible concentration (5 % en poids) forme avec la solution de polymère une dispersion homogène. La stabilité de la dispersion dépend des solvants ; ainsi la N-méthylpyrrolidone, qui est un solvant visqueux, conduit à une meilleure stabilité de la dispersion et les pertes en antimitotique par décantation ou adsorption sur les parois des béchers sont diminuées.

L'emploi de propane-1,2-diol comme agent de coacervation stabilise davantage le système en augmentant la viscosité du milieu.

**b) Rendement de production.**

Il varie de 15 à 100 % suivant les associations. Les associations utilisant l'acétate d'éthyle comme solvant du polymère donnent les meilleurs rendements, proches de 100 %.

**c) Taux et rendement d'encapsulation.**

Lorsque l'on augmente les charges théoriques en principe actif, le rendement d'encapsulation est très faible.

Un passage aux ultrasons permet d'individualiser les particules de principe actif. Lorsque l'on travaille avec de faibles concentrations, la dispersion est homogène et on atteint des rendements d'encapsulation de 70 %.

**Exemple 4: Etude de l'association N-méthylpyrrolidone/éthanol/eau + tensioactif.**

Parmi les polymères testés solubles dans la N-méthylpyrrolidone, on retiendra :

- Résomer® RG 502 (Boehringer Ingelheim), qui est un D,L-PLAGA 50 :50,
- Résomer® RG 756 (Boehringer Ingelheim) et Phusiline (Saint-Ismier) qui sont des D,L-PLAGA 75 : 25,

La formulation initiale des microparticules utilise une solution organique de polymère à 4 ou 10 % (p/v) pour un volume de solvant de 5 ml. La coacervation est réalisée sous agitation magnétique. Le non-solvant du polymère est ajouté à l'aide d'une micropipette, ml par ml. Dès l'apparition d'un trouble de la solution, un échantillon est prélevé et observé en microscopie optique. Une minute après l'apparition du trouble, le milieu de coacervation est versé goutte à goutte dans 200 ml de durcisseur (eau + Tween® 80 ou PVA). Le durcissement des microparticules est réalisé sous agitation mécantique. Une nouvelle observation en microscopie optique des microparticules est effectuée pendant le durcissement. Puis les microparticules sont filtrées sous vide, ou à pression atmosphérique sur papier filtre en cas d'impossibilité de filtration sous vide (colmatage quasi-immédiat du filtre ou durée de filtration trop importante). Enfin, les microparticules sont observées juste après filtration et après lyophilisation.

Les microparticules obtenues avec l'association NMP/éthanol/eau + tensioactif sont, en microscopie optique, sphériques, lisses et régulières, quel que soit le stade de la formulation et quel que soit le type de polymère.

Pour tous les lots réalisés, la filtration après durcissement a été réalisée sur papier filtre et à pression atmosphérique. La taille des microparticules est comprise entre 30 et 50 μm. De nombreuses microparticules d'environ 5 μm de diamètre sont aussi observées.

**• Influence du type de polymère et de sa concentration.**

Les microparticules obtenues présentent toutes le même aspect en microscopie optique.

Les meilleurs rendements sont obtenus avec Resomer® RG 502 et Resomer® RG 756 dans les conditions suivantes (tableau 3).

## TABLEAU 3

| Type de polymère | Concentration du polymère (p/v) | Type de durcisseur | Concentration du tensioactif dans le durcisseur (v/v) | Temps de durcissement | Température pendant la coacervation | Température pendant le durcissement | Rendement de fabrication |
|---|---|---|---|---|---|---|---|
| RG 502 | 10 % | PVA | 0,5 % | 3 h 30 | ambiante | ambiante | 23,8 % |
| RG 502 | 10 % | Tween® 80 | 1 % | 3 h 30 | ambiante | < 4°C | 35,9 % |
| RG 756 | 4 % | Tween® 80 | 1 % | 2 h | ambiante | ambiante | 49,0 % |
| RG 756 | 4 % | Tween® 80 | 5 % | 2 h | ambiante | ambiante | 38,6 % |

EP 1 216 091 B1

**• Influence de la nature du durcisseur et du temps de durcissement.**

Le type de durcisseur (eau, eau + Tween® 80, ou eau + PVA), pour le même polymère PLAGA 50 : 50 (RG 502) et à température ambiante, n'influe ni sur la filtration ni sur le rendement de fabrication des microparticules qui reste compris entre 17,5 et 23,8 %.
Le temps de durcissement, pour le même polymère PLAGA 50 : 50 (RG 502), permet d'augmenter le rendement de fabrication d'environ 10 %, à température ambiante.

**• Influence de la température**

a) Pendant le durcissement

Un durcissement à une température inférieure à 4°C a permis de doubler le rendement de fabrication des microparticules, pour le PLAGA 50 : 50 (RG 502) : 35,9 contre 17,5 %.

b) Pendant la coacervation

Les manipulations réalisées entièrement à une température inférieure à 4°C ont un rendement de fabrication de 10,5 % maximum.
L'association N-méthytpyrrotidone/éthanot/eau + tensioactif conduit à la formation de microparticules présentant un aspect satisfaisant en microscopie optique.

**Exemple 5 : Microsphères chargées avec la progestérone ou le budésonide.**

La progestérone et le budésonide sont des principes actifs hydrophobes solubles dans l'acétate d'éthyle.
On utilise l'association acétate d'éthyie/propan-2-ol/eau + Tween® 80, et le D,L-PLAGA 75 : 25 (Phusis, Saint-Ismier) comme polymère.
Les rendements de fabrication sont tous supérieurs à 90 %.
Les résultats obtenus sont rassemblés dans le tableau 4.

## TABLEAU 4

| Lot | Conc. polymère (p/v) et masse PA | Coacervation | | | Durcisseur | | | | | | Rende-ment de fabrica-tion | Granulo-métrie (µm) |
|---|---|---|---|---|---|---|---|---|---|---|---|---|
| | | Mode d'ajout du non-solvant | Tempé-rature | Agita-tion | Type | Conc. TA | Volume | Tempé-rature | Durée | Agitation | | |
| 1 | 4 % progestérone 28,5 mg (solution) | 200/200µl 1 min entre chaque ajout | - 4°C | Méca. 300 t/min | Tween® 80 | 10 % | 300 ml +150 ml +150 ml (filtr.) +300 ml | 0,5 °C | 1 h +30 min + 1 h (filt.) + 30 min | Méca. 500t/min | 96,7 % | 28,0 ± 12,5 |
| 2 | 4 % progestérone 30,1 mg (solution) | 200/200µl 1 min entre chaque ajout | - 4°C | Méca. 300 t/min | Tween® 80 | 10 % | 300 ml +150 ml +150 ml (filtr.) +300 ml | 0,5 °C | 1 h +30 min + 1 h (filt.) + 30 min | Méca. 500t/min | >99,9% | 25,3 ±9,2 |
| 3 | 4 % budésonide 30,8 mg (solution) | 200/200µl 1 min entre chaque ajout | - 4°C | Méca. 300 t/min | Tween® 80 | 10 % | 300 ml +150 ml +150 ml (filtr.) +300 ml | 0,5 °C | 1 h +30 min + 1 h (filt.) + 30 min | Méca. 500t/min | >99,9% | 20,8 ±8,7 |
| 4 | 4 % budésonide 29,8 mg (solution) | 200/200µl 1 min entre chaque ajout | - 4°C | Méca. 300 t/min | Tween® 80 | 10 % | 300 ml +150 ml +150 ml (filtr.) +300 ml | 0,5 °C | 1 h +30 min + 1 h (filt.) + 30 min | Méca. 500t/min | 97,6 % | 22,5 ±9,4 |

* filtration sous vide, filtre Nylon.

EP 1 216 091 B1

Les taux et rendements d'encapsulation des principes actifs figurent dans le tableau suivant et sont calculés comme suit :

- 

$$\text{Tthéo.} = [\text{poids de principe actif}/(\text{poids de polymère} + \text{poids de principe actif})] \times 100,$$

- 

$$\text{Texp.} = [\text{poids de principe actif}/\text{poids de microparticules sèches}] \times 100,$$

Ils permettent de calculer le rendement d'encapsulation R :

- 

$$R = [\text{Texp.}/\text{Tthéo.}] \times 100$$

| Taux et rendements d'encapsulation de la progestérone et du budésonide avec le PLAGA 75 : 25 (Phusis) | | | | |
|---|---|---|---|---|
| Lot | Taux Théorique | Taux expérimental | Rendement d'encapsulation | Taux d'humidité résiduelle |
| 1 (progestérone) | 12,4% | 8,2% | 66.1% | 11,9% |
| 2 (progestérone) | 13,1 % | 9,5 % | 72,5 % | - |
| 3 (budésonide) | 13,3 % | 8,0 % | 60,1 % | - |
| 4 (budésonide) | 13,0 % | 8,8 % | 67,7 % | 16,4 % |

L'association acétate d'éthyle/propan-2-ol/eau + tensioactif conduit à la formation de microparticules présentant un aspect satisfaisant en microscopie optique. En revanche, les microparticules obtenues avec ce couple solvant/non-solvant sont plus fragiles et sont séchées à l'air libre, donc à température ambiante et à pression atmosphérique, pour ne pas être agglomérées de façon irréversible. Les rendements de fabrication sont proches de 100 %. L'agrégation est le principal problème rencontré avec cette association solvant/non-so(vantldurcisseur. Elle peut être réduite en jouant sur différents paramètres de la formufation. La température à laquelle est effectuée la coacervation est notamment un facteur essentiel : abaissée à - 4°C, elle permet de figer le polymère et de rigidifier les microparticules qui, ainsi, s'individualisent et ne se collent plus entre elles.

**Revendications**

**1.** Procédé de microencapsulation d'un principe actif par coacervation qui consiste en

- la désolvatation ménagée ou coacervation d'un polymère dissous dans un solvant organique contenant ledit principe actif, ladite coacervation étant induite par addition d'un non-solvant et se traduisant par le dépôt du polymère à la surface du principe actif, puis
- le durcissement du dépôt de polymère par ajout d'un agent durcisseur, ledit durcissement se traduisant par la formation d'un film continu enrobant le principe actif,

**caractérisé en ce que**

- le solvant du polymère est un solvant organique non chloré de point d'ébullition compris entre 30°C et 240 °C et de permittivité diélectrique relative et comprise entre 4 et 60, avantageusement choisi parmi l'acétate d'éthyle, la N-méthylpyrrolidone, la méthyléthylcétone, l'acide acétique, le propylène carbonate et leurs mélanges,
- le non-solvant est un alcool ou une cétone comprenant 2 à 5 atomes, et préférentiellement 2 ou 3 atomes de carbone, en particulier l'éthanol ($\varepsilon=24$), le propan-2-ol ($\varepsilon=18$), le propane-1,2-diol ($\varepsilon$ entre 18 et 24) et le glycérol ($\varepsilon=40$), ou la méthyléthylcétone ($\varepsilon= 18$),
- l'agent durcisseur est choisi parmi l'eau, les alcools comprenant 1 à 4 atomes de carbone à condition que l'agent durcisseur soit un alcool différent du non-solvant, et leurs mélanges.

**2.** Procédé selon la revendication 1, **caractérisé en ce que** le non-solvant et le durcisseur sont respectivement choisis parmi les couples suivants : propane-1,2-diol et propan-2-ol, glycérol et propane-1,2-diol, glycérol et propan-2-ol, propan-2-ol et propane-1,2-diol.

**3.** Procédé selon les revendications 1 et 2, **caractérisé en ce que** le polymère est un polymère biodégradable dont la masse moléculaire en poids (Mw) est comprise entre 10 000 et 90 000 g/mole, de préférence entre 15 000 et 50 000 g/mole, dont l'indice de polydispemité (Ip) est compris entre 1 et 3,5, de préférence entre 1,5 et 2,5.

**4.** Procédé selon la revendication 3, **caractérisé en ce que** le polymère est un polymère d'acide lactique (PLA) ou un polymère d'acides lactique et glycolique (PLAGA).

**5.** Procédé selon la revendication 4, **caractérisé en ce que** le polymère est un PLAGA tel que Mw est comprise entra 15 000 et 25 000, de préférence égal à 17 500, Ip est compris entre 1 et 2, de préférence égal à 1,6, et le pourcentage d'acide glycolique est intérieur à 30%. de préférence égal à 25%.

**6.** Procédé selon l'une des revendications 1 à 5, **caractérisé en ce que** la concentration en polymère dans le solvant est comprise entre 1 et 10% (p/v), de préférence de l'ordre de 4% (p/v).

**7.** Procédé selon l'une des revendications précédentes, **caractérisé en ce que** le rapport volumique non-solvant/ solvant est compris entre 1/2 et 1/1.

**8.** Procédé selon l'une des revendications précédentes, **caractérisé en ce que** la température de coacervation est inférieure à la température de transition vitreuse du polymère, de préférence inférieure ou inférieure ou égale à 25°C, de préférence inférieure à 4°C, de préférence encore égale à -4°C.

**9.** Procédé selon l'une des revendications précédentes, **caractérisé en ce que** l'agent durcisseur contient en outre un agent tensioactif, la concentration dudit agent tensioactif dans l'agent durcisseur étant comprise entre 0,1 et 10% (v/v).

**10.** Procédé selon l'une des revendications précédentes, **caractérisés en ce que** l'agent tensioactif est un ester de sorbitane, par exempte le Polysorbate 80, ou l'alcool polyvinylique.

**11.** Procédé selon l'une des revendications précédentes, **caractérisé en ce que** le rapport volumique agent durcisseur/solvant est compris entre 5/1 et 180/1, et de préférence entre 15/1 et 120/1.

**12.** Procédé selon l'une des revendications précédentes, **caractérisé en ce que** le durcissement des microsphères est réalisé sous agitation à une vitesse comprise entre 500 et 1500 t/min.

**13.** Procédé selon l'une des revendications précédentes, **caractérisé en ce que** la température de durcissement est inférieure ou égale à 25°C, de préférence inférieure à 4°C, de préférence encore inférieure ou égale à 0,5°C.

**14.** Procédé selon l'une des revendications précédentes, **caractérisé en ce que** lorsque le principe actif forme une dispersion dans la solution de polymère, le solvant et le non-solvant ont une viscosité suffisamment élevée pour stabiliser le principe actif.

**15.** Procédé selon l'une des revendications précédentes, **caractérisé en ce que** le principe actif est dispersé sous ultra-sons pour former une dispersion dans la solution de polymère, et la coacervation est réalisée sous agitation douce, de préférence de type magnétique ou mécanique.

**16.** Procédé selon l'une des revendications précédentes, **caractérisé en ce que** la granulométrie du principe actif est comprise entre 1 et 50 micromètres, et préférentiellement entre 5 $\mu$m et 30 $\mu$m.

**17.** Procédé selon l'une des revendications précédentes, **caractérisé en ce que** le solvant est la N-méthylpyrrolidone, le non-solvant est l'éthanol, et le durcisseur est l'eau.

**18.** Procédé selon l'une des revendications 1 à 16, **caractérisé en ce que** le solvant est l'acétate d'éthyle.

**19.** Procédé selon la revendication 18, **caractérisé en ce que** le solvant est l'acétate d'éthyle, le non-solvant est le propan-2-ol, et le durcisseur est l'eau.

**20.** Procédé selon la revendication 18 ou 19, **caractérisé en ce que** le polymère est un PLAGA 75 :25 tel que Mw est comprise entre 15 000 et 20 000, de préférence égal à 17 500, Ip est compris entre 1 et 2, de préférence égal à 1,6.

**21.** Procédé selon l'une des revendications 1 à 16, **caractérisé en ce que** le solvant est l'acide acétique, le durcisseur est l'eau, et le polymère est un PLAGA 50 : 50.

**Claims**

**1.** Process for microencapsulating an active principle by coacervation, which consists of

- the controlled desolvation or coacervation of a polymer dissolved in an organic solvent containing the said active principle, the said coacervation being induced by addition of a nonsolvent and being reflected by the deposition of the polymer at the surface of the active principle, and then

- the curing of the polymer deposit by addition of a curing agent, the said curing being reflected by the formation of a continuous film coating the said active principle,

**characterized in that**

- the solvent for the polymer is a nonchlorinated organic solvent with a boiling point of between 30°C and 240°C and a relative dielectric permittivity of between 4 and 60, advantageously chosen from ethyl acetate, N-methylpyrrolidone, methyl ethyl ketone, acetic acid and propylene carbonate, and mixtures thereof,

- the nonsolvent is an alcohol or a ketone containing 2 to 5 carbon atoms and preferably 2 or 3 carbon atoms, in particular ethanol ($\varepsilon$=24), 2-propanol ($\varepsilon$=18), 1,2-propanediol ($\varepsilon$ between 18 and 24) and glycerol ($\varepsilon$=40), or methyl ethyl ketone ($\varepsilon$=18),

- the curing agent is chosen from water, alcohols containing 1 to 4 carbon atoms, on condition that the curing agent is an alcohol that is different than the nonsolvent, and mixtures thereof.

**2.** Process according to Claim 1, **characterized in that** the nonsolvent and the curing agent are chosen, respectively, from the following pairs: 1,2-propanediol and 2-propanol, glycerol and 1,2-propanediol, glycerol and 2-propanol, 2-propanol and 1,2-propanediol.

**3.** Process according to Claims 1 and 2, **characterized in that** the polymer is a biodegradable polymer with a weight molecular mass (Mw) of between 10,000 and 90,000 g/mol, preferably between 15,000 and 50,000 g/mol, and with a polydispersity index (Ip) of between 1 and 3.5 and preferably between 1.5 and 2.5.

**4.** Process according to Claim 3, **characterized in that** the polymer is a lactic acid polymer (PLA) or a polymer of lactic acid and of glycolic acid (PLAGA).

**5.** Process according to Claim 4, **characterized in that** the polymer is a PLAGA such that Mw is between 15,000 and 25,000, preferably equal to 17,500, Ip is between 1 and 2, and preferably equal to 1.6, and the percentage of glycolic acid is less than 30%, preferably equal to 25%.

**6.** Process according to one of Claims 1 to 5, **characterized in that** the polymer concentration in the solvent is between 1 and 10% (w/v) and preferably about 4% (w/v).

**7.** Process according to one of the preceding claims, **characterized in that** the nonsolvent/solvent ratio by volume is between 1/2 and 1/1.

**8.** Process according to one of the preceding claims, **characterized in that** the coacervation temperature is less than the glass transition temperature of the polymer, preferably less than or less than or equal to 25°C, preferably less than 4°C and more preferably equal to -4°C.

**9.** Process according to one of the preceding claims, **characterized in that** the curing agent also contains a surfactant, the concentration of the said surfactant in the curing agent being between 0.1 and 10% (v/v).

10. Process according to one of the preceding claims, **characterized in that** the surfactant is a sorbitan ester, for example Polysorbate 80 or polyvinyl alcohol.

11. Process according to one of the preceding claims, **characterized in that** the curing agent/solvent ratio by volume is between 5/1 and 180/1 and preferably between 15/1 and 120/1.

12. Process according to one of the preceding claims, **characterized in that** the microspheres are cured with stirring at a speed of between 500 and 1500 rpm.

13. Process according to one of the preceding claims, **characterized in that** the curing temperature is less than or equal to 25°C, preferably less than 4°C and more preferably less than or equal to 0.5°C.

14. Process according to one of the preceding claims, **characterized in that** when the active principle forms a dispersion in the solution for the polymer, the solvent and the nonsolvent have a viscosity that is high enough to stabilize the active principle.

15. Process according to one of the preceding claims, **characterized in that** the active principle is dispersed by ultrasound to form a dispersion in the polymer solution, and the coacervation is performed with gentle stirring, preferably of magnetic or mechanical type.

16. Process according to one of the preceding claims, **characterized in that** the particle size of the active principle is between 1 and 50 micrometres and preferably between 5 $\mu$m and 30 $\mu$m.

17. Process according to one of the preceding claims, **characterized in that** the solvent is N-methylpyrrolidone, the nonsolvent is ethanol and the curing agent is water.

18. Process according to one of Claims 1 to 16, **characterized in that** the solvent is ethyl acetate.

19. Process according to Claim 18, **characterized in that** the solvent is ethyl acetate, the nonsolvent is 2-propanol and the curing agent is water.

20. Process according to Claim 18 or 19, **characterized in that** the polymer is a 75:25 PLAGA such that the Mw is between 15,000 and 20,000 and preferably equal to 17,500, and the Ip is between 1 and 2 and preferably equal to 1.6.

21. Process according to one of Claims 1 to 16, **characterized in that** the solvent is acetic acid, the curing agent is water and the polymer is a 50:50 PLAGA.

**Patentansprüche**

1. Verfahren zur Mikroverkapselung eines Wirkstoffs durch Koazervation, welches besteht in:

   - der schonend vorgenommenen Desolvatation (Abtrennung vom Lösemittel) oder Koazervation eines in einem organischen Lösemittel, das den Wirkstoff enthält, gelösten Polymers, wobei die Koazervation durch Zugabe eines Nicht-Lösemittels induziert wird und durch die Ablagerung des Polymers auf der Oberfläche des Wirkstoffs zum Ausdruck kommt, dann
   - der Härtung der Polymerabscheidung durch Zugabe eines Härtungsmittels, wobei die Härtung durch die Bildung eines kontinuierlichen Films, welcher den Wirkstoff umhüllt, zum Ausdruck kommt,

   **dadurch gekennzeichnet, dass**

   - das Lösemittel des Polymers ein organisches, nichtchloriertes Lösemittel mit einem Siedepunkt zwischen 30°C und 240°C und einer relativen Dielektrizitätskonstante zwischen 4 und 60 ist, welches vorteilhafterweise unter Ethylacetat, N-Methylpyrrolidon, Methylethylketon, Essigsäure, Propylencarbonat und deren Mischungen ausgewählt wird,

- das Nicht-Lösemittel ein Alkohol oder ein Keton, welcher bzw. welches 2 bis 5 Kohlenstoffatome und vorzugsweise 2 oder 3 Kohlenstoffatome umfasst, insbesondere Ethanol ($\varepsilon$ = 24), Propan-2-ol ($\varepsilon$ = 18), Propan-1,2-diol ($\varepsilon$ zwischen 18 und 24) und Glycerol ($\varepsilon$ = 40) oder Methylethylketon ($\varepsilon$ = 18) ist,
- das Härtungsmittel unter Wasser, den 1 bis 4 Kohlenstoffatome enthaltenden Alkoholen mit der Maßgabe, dass das Härtungsmittel ein von dem Nicht-Lösemittel verschiedener Alkohol ist, und deren Mischungen ausgewählt wird.

2. Verfahren nach Anspruch 1, **dadurch gekennzeichnet, dass** das Nicht-Lösemittel und das Härtungsmittel jeweils unter den folgenden Paaren ausgewählt werden: Propan-1,2-diol und Propan-2-ol, Glycerol und Propan-1,2-diol, Glycerol und Propan-2-ol, Propan-2-ol und Propan-1,2-diol.

3. Verfahren nach den Ansprüchen 1 und 2, **dadurch gekennzeichnet, dass** das Polymer ein biologisch abbaubares Polymer ist, dessen auf das Gewicht bezogene Molekülmasse (Mw) zwischen 10000 und 90000 g/mol, vorzugsweise zwischen 15000 und 50000 g/mol liegt, dessen Polydispersitätsindex (Ip) zwischen 1 und 3,5, vorzugsweise zwischen 1,5 und 2,5 liegt.

4. Verfahren nach Anspruch 3, **dadurch gekennzeichnet, dass** das Polymer ein Milchsäurepolymer (PLA) oder ein Polymer von Milchsäure und Glycolsäure (PLAGA) ist.

5. Verfahren nach Anspruch 4, **dadurch gekennzeichnet, dass** das Polymer ein PLAGA ist, dessen Mw zwischen 15000 und 25000 liegt, vorzugsweise 17500 beträgt, dessen Ip zwischen 1 und 2 liegt, vorzugsweise 1,6 beträgt, und dessen Prozentsatz von Glycolsäure unter 30% liegt, vorzugsweise 25% beträgt.

6. Verfahren nach einem der Ansprüche 1 bis 5, **dadurch gekennzeichnet, dass** die Polymerkonzentration in dem Lösemittel zwischen 1 und 10% (Gew./Vol.), vorzugsweise in der Größenordnung von 4% (Gew./Vol.) liegt.

7. Verfahren nach einem der vorangegangenen Ansprüche, **dadurch gekennzeichnet, dass** das Volumenverhältnis Nicht-Lösemittel/Lösemittel zwischen 1/2 und 1/1 liegt.

8. Verfahren nach einem der vorangegangenen Ansprüche, **dadurch gekennzeichnet, dass** die Koazervationstemperatur unter der Glasübergangstemperatur des Polymers, vorzugsweise unter oder unter oder gleich 25°C, vorzugsweise unter 4°C liegt, noch mehr bevorzugt -4°C beträgt.

9. Verfahren nach einem der vorangegangenen Ansprüche, **dadurch gekennzeichnet, dass** das Härtungsmittel außerdem ein grenzflächenaktives Mittel enthält, wobei die Konzentration des grenzflächenaktiven Mittels in dem Härtungsmittel zwischen 0,1 und 10% (Vol./Vol.) liegt.

10. Verfahren nach einem der vorangegangenen Ansprüche, **dadurch gekennzeichnet, dass** das grenzflächenaktive Mittel ein Sorbitanester, beispielsweise Polysorbat 80, oder Polyvinylalkohol ist.

11. Verfahren nach einem der vorangegangenen Ansprüche, **dadurch gekennzeichnet, dass** das Volumenverhältnis Härtungsmittel/Lösemittel zwischen 5/1 und 180/1 und vorzugsweise zwischen 15/1 und 120/1 liegt.

12. Verfahren nach einem der vorangegangenen Ansprüche, **dadurch gekennzeichnet, dass** die Härtung der Mikrokugeln unter Rühren mit einer Geschwindigkeit zwischen 500 und 1500 U/min erfolgt.

13. Verfahren nach einem der vorangegangenen Ansprüche, **dadurch gekennzeichnet, dass** die Härtungstemperatur unter oder gleich 25°C, vorzugsweise unter 4°C, noch mehr bevorzugt unter oder gleich 0,5°C beträgt.

14. Verfahren nach einem der vorangegangenen Ansprüche, **dadurch gekennzeichnet, dass**, wenn der Wirkstoff eine Dispersion in der Polymerlösung bildet, das Lösemittel und das Nicht-Lösemittel eine ausreichend hohe Viskosität haben, um den Wirkstoff zu stabilisieren.

15. Verfahren nach einem der vorangegangenen Ansprüche, **dadurch gekennzeichnet, dass** der Wirkstoff unter Ultraschall dispergiert wird, um eine Dispersion in der Polymerlösung zu bilden, und die Koazervation unter sanfter Bewegung, vorzugsweise vom magnetischen oder mechanischen Typ, ausgeführt wird.

16. Verfahren nach einem der vorangegangenen Ansprüche, **dadurch gekennzeichnet, dass** die Korngrößenverteilung des Wirkstoffs zwischen 1 und 50 Mikrometern und vorzugsweise zwischen 5 µm und 30 µm liegt.

17. Verfahren nach einem der vorangegangenen Ansprüche, **dadurch gekennzeichnet, dass** das Lösemittel N-Methylpyrrolidon ist, das Nicht-Lösemittel Ethanol ist und das Härtungsmittel Wasser ist.

18. Verfahren nach einem der Ansprüche 1 bis 16, **dadurch gekennzeichnet, dass** das Lösemittel Ethylacetat ist.

19. Verfahren nach Anspruch 18, **dadurch gekennzeichnet, dass** das Lösemittel Ethylacetat ist, das Nicht-Lösemittel Propan-2-ol ist und das Härtungsmittel Wasser ist.

20. Verfahren nach Anspruch 18 oder 19, **dadurch gekennzeichnet, dass** das Polymer ein solches PLAGA 75:25 ist, dass dessen Mw zwischen 15000 und 20000 liegt, vorzugsweise 17500 beträgt, dessen Ip zwischen 1 und 2 liegt, vorzugsweise 1,6 beträgt.

21. Verfahren nach einem der Ansprüche 1 bis 16, **dadurch gekennzeichnet, dass** das Lösemittel Essigsäure ist, das Härtungsmittel Wasser ist und das Polymer ein PLAGA 50:50 ist.